# EUROPEAN PATENT SPECIFICATION

(11) **EP 4 308 247 B1**
(45) Date of publication and mention of the grant of the patent: **27.11.2024**
(21) Application number: 22712416.1
(22) Date of filing: 11.03.2022
(51) Int. Cl.: A61Q 19/08, A61K 8/37

(54) **USE OF MONOGLYCERIDES FOR TREATING SIGNS OF SKIN AGING**
VERWENDUNG VON MONOGLYCERIDEN ZUR BEHANDLUNG VON ANZEICHEN DER HAUTALTERUNG
UTILISATION DE MONOGLYCÉRIDES POUR TRAITER LES SIGNES DE VIEILLISSEMENT DE LA PEAU

(30) Priority: 16.03.2021 EP 21162784
(43) Date of publication of application: 24.01.2024
(73) Proprietor: Unilever IP Holdings B.V., 3013 AL Rotterdam (NL); Unilever Global IP Limited, Wirral, Merseyside CH62 4ZD (GB)
(72) Inventor: BISHOP, Cleo, Lucinda, 6708 WH Wageningen (NL); GUNN, David, Andrew, 6708 WH Wageningen (NL); HARICHIAN, Bijan, deceased (NL); JENKINS, Gail, 6708 WH Wageningen (NL); LATHROP, William, F., 6708 WH Wageningen (NL); MILLIGAN, Deborah, Anne, 6708 WH Wageningen (NL); ROSA, Jose, Guillermo, 6708 WH Wageningen (NL); TYLER, Eleanor, Jane, 6708 WH Wageningen (NL); WAINWRIGHT, Linda, Jane, 6708 WH Wageningen (NL)
(74) Representative: James, Helen Sarah
(86) International application number: PCT/EP2022/056408
(87) International publication number: WO 2022/194718

(56) References cited:
- CN-A- 107 174 534
- JP-A- H10 152 429

## Description

### Field of the Invention

The present invention relates to the use of monoglycerides for treating signs of skin aging.

### Background of the Invention

With ever-rising life expectancies around the world, the physical appearance of aging is becoming an increasingly common cosmetic issue. Skin appearance plays a particularly crucial role for self-esteem and social interactions, and skin health and beauty is considered one of the principal factors representing overall well-being and the perception of health in humans, Therefore, the demand for cosmetic products that may be applied topically to the skin to combat skin aging, especially facial skin aging, has grown enormously.

Skin aging is a complex process, and alterations in human skin due to aging have distinct characteristics as compared to other organs. Characteristics of intrinsic or chronological skin aging include dryness, visible fine lines and wrinkles, uneven skin pigmentation, loss of elasticity and skin sagging. Extrinsic factors including exposure to sunlight, pollutants and cigarette smoke can accelerate the skin aging process, especially on the face.

Cellular senescence has been correlatively linked to the aging process at the level of the whole animal, and senescence of skin cells, especially dermal fibroblasts, is thought to be a significant driver of the aging-related phenotype in skin. It is therefore highly desirable to develop interventions which can target skin aging at the cellular level by selectively eliminating senescent skin cells, particularly senescent dermal fibroblasts.

The present invention addresses this problem.

### Summary of the Invention

The present invention provides the cosmetic use, as an active ingredient for treating signs of skin aging, of one or more monoglycerides of general formula (I):

RC(O)-X (I)

in which R is a linear or branched, saturated or unsaturated, hydrocarbon chain which contains from 12 to 22 carbon atoms and which is optionally substituted with one or more hydroxyl substituents; and X is a glyceryl residue.

The invention also provides the use of one or more monoglycerides of general formula (I) as an active ingredient in, and for the manufacture of, topical compositions for treating signs of skin aging.

The invention also provides one or more monoglycerides of general formula (I) as an active ingredient for use in the selective elimination of senescent skin cells.

The invention also provides the use of one or more monoglycerides of general formula (I) as an active ingredient in, and for the manufacture of, topical compositions for the selective elimination of senescent skin cells.

The invention also provides a cosmetic method for treating signs of skin aging, comprising topically applying to the skin a cosmetic composition containing one or more monoglycerides of general formula (I).

### Detailed Description and Preferred Embodiments

As used herein, "cosmetic" refers to a treatment which does not cure, treat or prevent a disease or disorder, but instead serves as a skincare product or to modify or improve the appearance of the skin, e.g. the colour, texture or moisture content of the skin.

### Monoglycerides

Monoglycerides of general formula (I) as defined above can be classified into two types, depending on the position of the RC(O)- group: 1-monoglycerides (a-monoglycerides), in which the RC(O)- groups are attached to the C₁ or C₃ atom of glycerol; and 2-monoglycerides (β-monoglycerides) in which the RC(O)- groups are attached to the C₂ atom of glycerol.

Monoglycerides may be produced industrially from the glycerolysis or hydrolysis of triglycerides, or the direct esterification of glycerol with fatty acids. The most common method for production of monoglycerides is by glycerolysis of natural or hydrogenated fats and/or oils, such as those obtained from coconut, palm, palm kernel, soya, rapeseed (canola), sunflower, cottonseed, corn, olive, tallow and lard. This generally yields a mixture containing about 35 to 50% (by weight based on total weight) monoglycerides, together with variable quantities of diglycerides, unreacted triglycerides, residual glycerol and free fatty acids. Vacuum or molecular distillation may suitably be applied to the mixture to obtain a final product containing from 90 to 100% (by weight based on total weight) monoglycerides.

In preferred monoglycerides of general formula (I) for use in the invention, R is selected from linear, saturated or monounsaturated, hydrocarbon chains which contain from 14 to 20 carbon atoms and 0 or 1 hydroxyl substituents. More preferably R is selected from linear saturated hydrocarbon chains which contain from 16 to 18 carbon atoms and 0 or 1 hydroxyl substituents. A most preferred monoglyceride for use in the invention is glyceryl monostearate. Glyceryl monostearate is also known by the following technical names: glyceryl stearate, monostearin, glycerol stearate, stearic acid monoglyceride; and 1,3-dihydroxypropan-2-yl octadecanoate 2,3-dihydroxypropyl octadecanoate.

Mixtures of any of the above described materials may also be used.

According to this invention, the one or more monoglycerides as defined above are used for treating signs of skin aging.

In the context of this invention, the term "treating" denotes reducing, preventing or eliminating. The expression "signs of skin aging" denotes one or more characteristics of intrinsic or chronological aging such as thin and dry skin, fine wrinkles, decreased elasticity and aberrant pigmentation. These signs affect everyone, whatever their skin type or state of health. As noted above, the process may be amplified by extrinsic factors such as exposure to sunlight, pollutants and cigarette smoke.

Signs of skin aging can be measured using clinical and biophysical methods. Functional parameters like skin surface pH, stratum corneum hydration (SCH) or transepidermal water loss (TEWL) are for instance applied as markers of the status and integrity of the skin barrier. During aging, skin surface pH may increase, whereas TEWL decreases. Structural assessments such as skin microtopography and Cutometer^{®} measurements may be used to measure surface roughness and skin elasticity respectively. During aging, surface roughness may increase, whereas skin elasticity decreases. Age-dependent skin colour changes, such as the degree of luminance and yellowness in the skin tone, may be measured using spectrophotometry and expressed as L* and b* respectively in the CIE *L** *a** b* colour space. In addition to these instrumental measures, clinical skin aging scales are used in skin research and aesthetic dermatology. These scales are generally based on skilled assessment of skin dyspigmentation, wrinkling and sagging. Assessment is performed on standardized photographs taken from the test subject's face from a front and 45° right and 45° left view.

According to this invention, the one or more monoglycerides of general formula (I) are used in the selective elimination of senescent skin cells.

In the context of this invention, the term "senescent" refers to a state of permanent cell cycle arrest in which cells remain metabolically active and adopt characteristic phenotypic changes. The establishment of this phenotype is believed to be either the result of telomere shortening after several cell divisions (replicative senescence) or a response to stress stimuli (stress-induced senescence). One of the defining features of senescent cells is their stable cell cycle arrest. This cell cycle exit is controlled by activation of the p53/p21^{CIP1} and p16^{INK4a}/Rb tumour suppressor pathways. Unlike quiescent cells, senescent cells are nonresponsive to mitogenic or growth factor stimuli; thus, they are unable to reenter the cell cycle even in advantageous growth conditions. Senescent cells are also distinct from terminally differentiated cells, which are also irreversibly withdrawn from cell cycle. While terminal differentiation is the result of a defined developmental programme, which turns undifferentiated precursors into specialized effector cells, senescence is mainly implemented as a cellular stress response.

Senescent cells can be identified *in vitro* and *in vivo,* since they display a number of characteristics that allow their identification.

For example, senescent cells often appear multinucleated, large and extended, and exhibit spindle and vacuolisation features. They also display modifications in the organisation of chromatin that can help identify them. In normal cells, DNA staining reveals completely uniform colour outlines, whereas senescent cells usually show dot-like patterns, known as senescence-associated heterochromatic foci (SAHF). This phenomenon is due to intensive remodelling in the chromatin, which results in less susceptibility for digestion by nucleases.

Furthermore, senescence-related chromatin remodelling leads to profound transcriptional changes. Among the assortment of upregulated genes is a prominent subset of genes that encode secreted proteins, including cytokines and chemokines with proinflammatory properties, as well as various growth factors and proteases that together alter tissue structure and function. Collectively, these factors are referred to as the senescence-associated secretory phenotype (SASP). The SASP is one of the key characteristics that distinguish senescent cells from quiescent, terminally differentiated, and other types of non-proliferating cells.

A distinctive measurable feature of senescent cells is the presence of β-galactosidase enzymatic activity. This enzyme normally displays activity at pH 4.0 within lysosomes, but in senescent cells it is also active at pH 6.0. This phenomenon is termed senescence associated-β-galactosidase (SA-β-gal) activity and is thought to be due to an enlargement in the structure of lysosomes in senescent cells. SA-β-gal activity is detectable by histochemical staining by using X-gal as a substrate for SA-β-gal. Since SA-β-gal activity is detected in most senescent settings, both *in vitro* and *in vivo,* it is considered a de facto hallmark of senescence.

Selective elimination of senescent cells may be determined by measuring a viability ratio of senescent cells versus proliferating cells, when populations of the respective cell types are each incubated with a fixed concentration of a test compound in a suitable medium.

Compounds may be determined as capable of selectively eliminating senescent cells if the calculated viability ratio of the senescent cells versus the proliferating cells is lower than 0.9, preferably lower than 0.75.

Selective elimination of senescent cells may also be determined by incubating populations of senescent cells and proliferating cells respectively with various concentrations of a test compound in a suitable medium and measuring the LD50 value in each case (i.e. the concentration of the compound required to kill 50% of the cells in the respective population).

Compounds may be determined as capable of selectively eliminating senescent cells if the LD50 of the compound in proliferating cells is at least 2 times higher, preferably at least 3 times higher, than the LD50 of the compound in senescent cells.

As used herein, the term "skin cells" may refer to any skin resident cell type (such as dermal fibroblasts, melanocytes and epidermal keratinocytes), or cell types from skin appendages (such as sebocytes), or a mixture thereof. Preferably the term refers to dermal fibroblasts, especially dermal papillary fibroblasts.

In any of the method and uses described herein, the one or more monoglycerides of general formula (I) can be used *per se* or formulated with other ingredients to make a topical cosmetic or dermatological composition.

### Product Forms

Topical compositions comprising the one or more monoglycerides of general formula (I) for use in the invention can be formulated in a variety of forms for topical application, and will generally contain from about 0.01% to about 10%, preferably from about 0.1% to 1% by weight of the one or more monoglycerides of general formula (I).

Topical compositions for use in the invention are preferably cosmetic in nature and will generally include a cosmetically acceptable vehicle. The term "cosmetically acceptable" means that the vehicle is suitable for topical application to the skin, has good aesthetic properties, is compatible with the one or more monoglycerides of general formula (I) and any other ingredients, and will not cause any safety or toxicity concerns.

The vehicle may comprise an aqueous phase, an oil phase, an alcohol, a silicone phase or a mixture thereof, and may be in the form of an emulsion. Emulsions can have a range of consistencies including thin lotions (which may also be suitable for spray or aerosol delivery), creamy lotions, light creams and heavy creams.

Exemplary emulsions include water-in-oil emulsions, oil-in-water emulsions, silicone-in-water emulsions, water-in-silicone emulsions, polyol-in-silicone emulsions, silicone-in-polyol emulsions, polyol-in-oil emulsions, oil-in-polyol emulsions, wax-in-water emulsions and water-oil-water triple emulsions. Preferred emulsions include oil-in-water emulsions and water-in-oil emulsions.

Topical cosmetic compositions in the form of an emulsion, and suitable for use in the invention, typically have an oil phase containing at one or more cosmetically acceptable fatty materials which may be liquid or solid at room temperature (25°C).

Suitable cosmetically acceptable fatty materials include naturally derived oils (such as sunflower oil, borage oil, soybean oil, castor oil, olive oil and almond oil); esters of monoalcohols or of glycols with monocarboxylic or polycarboxylic acids, at least one of the alcohols and/or acids comprising at least one hydrocarbon-based chain containing at least 6 carbon atoms (such as octyl palmitate, isopropyl myristate, isopropyl palmitate, isopropyl isostearate, hexyl laurate, isohexyl laurate, isohexyl palmitate, decyl oleate, isodecyl oleate, hexadecyl stearate, decyl stearate, dihexyldecyl adipate, lauryl lactate, myristyl lactate, cetyl lactate, oleyl stearate, oleyl oleate, oleyl myristate, lauryl acetate, cetyl propionate, isononyl isononanoate, propylene glycol dicaprate, diisopropyl adipate, dibutyl adipate, and oleyl adipate); ethers (such as dicapryl ether); fatty alcohols (such as cetyl alcohol, stearyl alcohol and behenyl alcohol); isoparaffins (such as isooctane, isododecane and isohexadecane); silicone oils (such as dimethicones, cyclic silicones, and polysiloxanes); and hydrocarbon oils (such as mineral oil, petrolatum and polyisobutene); fatty acids containing from 8 to 30 carbon atoms, (such as stearic acid, lauric acid, palmitic acid and oleic acid); vegetable fats (such as cocoa butter, coconut oil, palm oil and shea butter) ; natural or synthetic waxes (such as lanolin wax, beeswax, carnauba wax, candelilla wax, paraffin wax, lignite wax, microcrystalline waxes, ceresin, ozokerite, and polyethylene waxes), hydrogenated oils which are solid at 25° C (such as hydrogenated castor oil, hydrogenated jojoba oil, hydrogenated palm oil, hydrogenated tallow and hydrogenated coconut oil) and fatty esters that are solid at 25°C (such as C₂₀₋₄₀ alkyl stearate).

Mixtures of any of the above described materials may also be used.

Topical cosmetic compositions in the form of an emulsion, and suitable for use in the invention, typically have an aqueous phase, which may also include one or more organic liquids that are miscible with water at room temperature (25°C). Exemplary water-miscible organic liquids include monohydric and polyhydric alcohols and derivatives thereof such as C₂-C₆ alkanols (such as ethanol and isopropanol); C₂-C₁₀ glycols and polyols (such as glycerol, propylene glycol, butylene glycol, pentylene glycol, hexylene glycol, caprylyl glycol, dipropylene glycol, and diethylene glycol); C₃-C₁₆ glycol ethers (such as mono-, di-, or tripropylene glycol (C₁-C₄) alkyl ethers and mono-, di-, or triethylene glycol (C₁-C₄) alkyl ethers) and polyethylene glycol having 2 to 12 oxyethylene units.

Topical cosmetic compositions in the form of an emulsion, and suitable for use in the invention, generally include surface active ingredients, such as emulsifiers and solubilizers, to enable two or more immiscible components to be combined homogeneously and to help stabilize the composition. Emulsifiers that may be used to form O/W or W/O emulsions include sorbitan oleate, sorbitan sesquioleate, sorbitan isostearate, sorbitan trioleate, PEG-20 sorbitan isostearate, polyglyceryl-3-diisostearate, polyglycerol esters of oleic/isostearic acid, polyglyceryl-6 hexaricinolate, polyglyceryl-4-oleate, polyglyceryl-4 oleate/PEG-8 propylene glycol cocoate, polyglyceryl-2 dipolyhydroxystearate, PEG-30 dipolyhydroxystearate, oleamide DEA, TEA myristate, TEA stearate, magnesium stearate, sodium stearate, potassium laurate, potassium ricinoleate, sodium cocoate, sodium tallowate, potassium castorate, sodium oleate, cetyl phosphate, diethanolamine cetyl phosphate, potassium cetyl phosphate, sodium glyceryl oleate phosphate, dimethicone copolyol, cetyl dimethicone copolyol, octyldimethicone ethoxyglucoside copolyol, dimethicone copolyol crosspolymer and laurylmethicone copolyol.

Mixtures of any of the above described materials may also be used.

Topical cosmetic compositions for use in the invention may also be formulated in a single-phase carrier such as water and/or one or more water miscible organic liquids (such as the monohydric and polyhydric alcohols and derivatives thereof described above).

Topical cosmetic compositions for use in the invention may also be formulated in solid forms such as gels or sticks.

Combinations of any of the above described product forms may also be used.

### Skin Care Actives

In addition to the one or more monoglycerides of general formula (I), a topical cosmetic composition for use in the invention may include additional skin care actives (for improving the physical and/or aesthetic characteristics of the skin). Examples of additional skin care actives which are suitable for use in the invention include vitamins, minerals and/or antioxidants, emollients, humectants, skin anti-hyperpigmentation agents, sunscreens, anti-irritants, exfoliating agents and mixtures thereof.

Vitamins, minerals and/or antioxidants suitable for use in the invention include natural botanical antioxidants derived from plant materials such as fruits, vegetables, herbs and spices (such as goji berry, white tea, rosemary, green tea, grape seed and lemongrass extracts); vitamin A and its precursors or derivatives (such as beta-carotene, retinyl palmitate); vitamin B3 and its precursors or derivatives (such as niacinamide); vitamin B5 and its precursors or derivatives (such as panthenol and its precursors or derivatives); vitamin C and its precursors or derivatives (such as tetrahexyldecyl ascorbate, ascorbyl palmitate); vitamin E and its precursors or derivatives (such as d-alpha-tocopherol, tocopheryl acetate); vitamin K and its precursors or derivatives; selenium and its derivatives (such as L-selenomethionine); and alpha lipoic acid.

Emollients suitable for use in the invention act to increase and maintain moisture in the skin by providing a protective coating to impede epidermal water loss. Examples of emollients include C₁₀₋₂₀ fatty alcohols and acids (such as cetyl, myristyl, palmitic and stearyl alcohols and acids); and C₁₀₋₄₀ hydrocarbons (such as mineral oil, petroleum jelly, squalene and isoparaffins).

Humectants suitable for use in the invention act to increase and maintain moisture in the skin by attracting water to the stratum corneum of the epidermis. Examples of humectants include amino acids, chondroitin sulfate, glycerin, diglycerin, triglycerin, polyglycerin, polypropylene glycol, polyethylene glycol, ethylene glycol, diethylene glycol, triethylene glycol, propylene glycol, dipropylene glycol, hexylene glycol, 1,3-butylene glycol, 1,4-butylene glycol, ethylene glycol monoalkyl ether, diethylene glycol monoalkyl ether, erythritol, fructose, glucose, maltose, sucrose, lactose, xylose, inositol, lactitol, xylitol, sorbitol, mannitol, maltitol, panthenol, pentaerythritol, 1,2,6-hexanetriol, honey, hyaluronic acid, hydrogenated honey, hydrogenated starch hydrolysate, natural moisturization factor, PEG-15 butanediol, polyglyceryl sorbitol, sodium and potassium salts of pyrollidone carboxylic acid, sodium glucuronate, trehalose and urea.

Skin anti-hyperpigmentation agents suitable for use in the invention include natural botanical agents derived from plant materials (such as *Arctostaphylos patula* and *Arctostaphylos viscida* extracts, *Emblica officinalis* extract, *Mitracarpus scaber* extract, *Uva ursi* (bearberry) extract, *Morus bombycis* (mulberry) extract, *Morus alba* (white mulberry) extract, *Broussonetia papyrifera* (paper mulberry) extract, licorice extract, acerola cherry extract, *Chlorella vulgaris* extract, *Aloe ferrox* extract and *Rumex occidentalis* extract); synthetic or natural sugar amines (such as glucosamine, N-acetyl glucosamine, glucosamine sulfate, mannosamine, N-acetyl mannosamine, galactosamine, N-acetyl galactosamine and their hydrochloride salts); retinoids (such as retinol, retinal, retinoic acid and C₂₋₂₀ esters of retinol such as retinyl palmitate, retinyl acetate, retinyl propionate, retinyl linoleate and retinyl oleate); kojic acid and C₂₋₂₀ esters thereof (such as kojic acid monobutyrate, kojic acid monocaprate, kojic acid monopalmitate, kojic acid monostearate and kojic acid monobenzoate); hydroquinone, hydroquinone monomethyl ether, hydroquinone monoethyl ether, hydroquinone monobenzyl ether, α and β-arbutin, deoxyarbutin (4-[(tetrahydro-2H-pyran-2-yl)oxy]phenol), mequinol (4-hydroxyanisole), glutathione, cysteine, N-acetyl-L-cysteine, azelaic acid, magnesium ascorbyl phosphate, sodium ascorbyl phosphate, peroxides (such as hydrogen peroxide, zinc peroxide, sodium peroxide and benzoyl peroxide); 3-aminotyrosine, glycyrrhizinic acid and 4-substituted resorcinol derivatives (such as 4-methyl resorcinol, 4-ethyl resorcinol, 4-propyl resorcinol, 4-isopropyl resorcinol, 4-butyl resorcinol, 4-pentyl resorcinol, 4-hexyl resorcinol, 4-heptyl resorcinol, 4-octyl resorcinol, 4-nonyl resorcinol, 4-decyl resorcinol, 4-undecyl resorcinol, 4-dodecyl resorcinol, 4-cyclopentyl resorcinol, 4-cyclohexyl resorcinol, 4-cycloheptyl resorcinol, and 4-cyclooctyl resorcinol).

Sunscreens suitable for use in the invention protect the skin from ultraviolet (UV) solar radiation falling within both the UVB region (between 290nm to 320 nm wavelengths) and the UVA region (between 320nm and 400nm wavelengths). Examples of sunscreens include methoxycinnamate derivatives (such as octyl methoxycinnamate and isoamyl methoxycinnamate); camphor derivatives (such as 4-methyl benzylidene camphor, camphor benzalkonium methosulfate, and terephthalylidene dicamphor sulfonic acid); salicylate derivatives (such as octyl salicylate and homosalate); sulfonic acid derivatives (such as phenylbenzimidazole sulfonic acid); benzone derivatives (such as dioxybenzone, sulisobenzone, and oxybenzone); benzoic acid derivatives (such as aminobenzoic acid and octyldimethyl para-amino benzoic acid); octocrylene, diethylhexyl butamido triazone, octyl triazone, butyl methoxydibenzoyl methane, drometrizole trisiloxane, menthyl anthranilate and inorganic UV-absorbing particles (such as zinc oxide and titanium dioxide).

Anti-irritants suitable for use in the invention include allantoin, aloe vera, α-bisabolol, caffeine, chamomile extract, *Cola nitada* extract, cucumber extract, dipotassium glycyrrhizinate, glycyrrhizic acid, green tea extract, lecithin or hydrogenated lecithin, licorice extract, tea tree oil, salicylic acid, acetylsalicylic acid, strontium acetate, strontium chloride, strontium nitrate, fatty acids with anti-irritant properties (such as linoleic acid and linolenic acid) and aromatic aldehydes with anti-irritant properties (such as 4-methoxy benzaldehyde, 4-ethoxy benzaldehyde, 4-butoxy benzaldehyde and 4-pentoxy benzaldehyde).

Exfoliating agents suitable for use in the invention include benzoyl peroxide, benzoic acid, 3-hydroxy benzoic acid, salicylic acid, acetic acid, trichloroacetic acid, 1-pyrrolidone-5-carboxylic acid, α-hydroxy acids (such as glycolic acid, lactic acid, malic acid, tartaric acid, and citric acid); β-hydroxy acids (such as β-hydroxybutyric acid); α-keto acids (such as pyruvic acid, 2-oxopropanoic acid, 2-oxobutanoic acid and 2-oxopentanoic acid); and oxa acids (such as 3,6,9-trioxaundecanedioic acid).

Mixtures of any of the above described materials may also be used.

### Functional ingredients

In addition to the one or more monoglycerides of general formula (I), a topical cosmetic composition for use in the invention may include additional functional ingredients (for improving the physical and/or aesthetic characteristics of the composition).

Examples of additional functional ingredients which are suitable for use in the invention include water soluble or colloidally water soluble polymeric thickening agents (such as hydroxyethyl cellulose, methyl cellulose, hydroxypropyl methyl cellulose, polyquaternium-10, carrageenan, guar gum, hydroxypropyl guar gum, xanthan gum, polyvinylalcohol, acrylic acid/ethyl acrylate copolymers, carboxyvinyl polymers, cross-linked polyacrylate polymers and polyacrylamide polymers); structurant clays (such as magnesium aluminum silicate, attapulgite, bentonite, montmorillonite and hectorite); inorganic pigments (such as titanium oxide, zirconium oxide, cerium oxide zinc oxide, iron oxide, chromium oxide and ferric blue); organic pigments (such as carbon black and barium, strontium, calcium, and aluminium lakes); pearlescent agents (such as mica coated with titanium oxide and/or iron oxide); dyes, preservatives (such as disodium EDTA, benzyl alcohol, methylparaben, phenoxyethanol, propylparaben, ethylparaben, butylparaben and isobutylparaben); pH adjusters and fragrances.

Mixtures of any of the above described materials may also be used.

### Packaging and Use

A topical cosmetic composition for use in the invention (as described above) may be packaged in a suitable container to suit its viscosity and intended use by the consumer. For example, a lotion or a cream can be packaged in a bottle or a roll-ball applicator, or a propellant-driven aerosol device or a container fitted with a pump suitable for finger operation. When the composition is a cream, it can simply be stored in a non-deformable bottle or squeeze container, such as a tube or a lidded jar.

The composition is suitably applied to the skin, preferably facial skin, at the rate of one or two applications per day. Generally, an amount corresponding to about 1 to 2 ml of the composition per application is applied uniformly over the area of treatment twice daily for a period of at least 7 (seven) days, more preferably at least 30 (thirty) days.

The invention will be further illustrated by the following, non-limiting Example.

### EXAMPLE

A high-throughput compound screen of 1240 test compounds including *inter alia* monostearin (IUPAC: 1,3-dihydroxypropan-2-yl octadecanoate 2,3-dihydroxypropyl octadecanoate) was optimised in primary adult human dermal fibroblasts (HDFs). For this, a model of replicative senescence in HDFs was established. In brief, early proliferating (EP) fibroblasts at passage 11 were serially passaged until they reached replicative senescence at passage 29. Deeply senescent (DS) fibroblasts were then defined as a population which did not expand when kept in culture for three weeks post-replicative senescence.

Actinomycin D, a drug widely reported to induce apoptosis in multiple cell types, was optimised for use as a positive 'senolytic control' for the high-throughput compound screen in EP (0.02µM) and DS (0.2µM) HDFs.

In order to perform the high-throughput screen of the test compounds a thorough validation process of the assay was undertaken prior to experimental screening. For both EP and DS HDFs, the following steps were optimised: optimisation of cell seeding densities in 384-well plate format; validation and optimisation of Actinomycin D positive and DMSO-vehicle negative controls; optimisation of high-throughput image acquisition and high content analysis of Hoechst 33342 (ThermoFisher, UK) in combination with SYTOX Green (ThermoFisher, UK) using the INCell 2200 microscope (GE, UK) for cell death phenotypic read-outs; optimisation of liquid handling system protocols; plate uniformity assessments; and a "dry run" of all components in the assay.

Following successful optimisation of high-throughput test compound screening, P7 and P39+1 HDFs were thawed, passaged or kept in culture, respectively, and manually seeded at P9 (EP) or P39+3 (DS) into 384-well plate format on day fourteen. On day fifteen, a liquid handling robot added test compounds to the cells at three serial concentrations. The negative DMSO vehicle control and Actinomycin D positive control (0.02µM for EP HDFs and 0.2µM for DS HDFs) were manually added to each plate. Subsequently, cells were incubated with the test compounds for 48 hours before media changing on day seventeen. At five days post- test compound addition, plates were incubated with a combination of Hoechst 33342 and SYTOX Green, fixed, imaged using the INCell 2200 automated microscope, and cell number, SYTOX % and nuclear area quantified using the IN Cell Developer software 1.9.1 (GE, UK). The screening of all 1240 test compounds at three serial concentrations was performed once with three technical replicates for EP and two technical replicates for DS HDFs.

Following successful high-throughput compound screening of the test compounds in both EP and DS HDFs, a number of post-screen quality control checks were employed prior to hit detection. First, negative and positive control inter- and intra-plate cell number variability was assessed in order to identify potential systemic issues. Furthermore, replicate variability was assessed, and any variability between replicates used to identify potential false positive out-of-focus wells which were subsequently re-imaged and re-analysed.

Lastly, the data was thresholded for hit identification based on the variability of negative controls. Briefly, the minimum negative control value across all plates was divided by the mean of all negative control values which generated a cell viability threshold for each EP and DS screen (89% EP HDFs; 87% DS HDFs). For scoring a positive hit (or `senolytic'), the following highly stringent cut-offs were assigned: EP cell viability above the 89% EP threshold and DS cell viability below the 87% DS threshold for at least one concentration of the three serial concentrations. Conversely, test compounds which decreased EP cell viability below the 89% EP threshold but did not decrease DS cell viability below 87% were termed `prolifolytic'. Test compounds which decreased both EP and DS cell viability below their respective thresholds were termed 'cytotoxic' and test compounds which maintained both EP and DS viability above their respective thresholds were defined as 'non-toxic'.

The results showed that monostearin according to the invention was defined as 'senolytic' for at least one concentration of the three serial concentrations in the EP and DS HDFs.

Therefore, monostearin according to the invention demonstrates a preferential cytotoxicity for senescent cells, compared to growing cells.

## Claims

1. Cosmetic use of one or more monoglycerides of general formula (I):
RC(O)-X (I)
in which R is a linear or branched, saturated or unsaturated, hydrocarbon chain which contains from 12 to 22 carbon atoms and which is optionally substituted with one or more hydroxyl substituents; and X is a glyceryl residue; as an active ingredient for treating signs of skin aging by selectively eliminating senescent skin cells.

## Patentansprüche

1. Kosmetische Verwendung eines oder mehrerer Monoglyceride der allgemeinen Formel (I):
RC(O)-X (I),
in welcher R eine lineare oder verzweigte, gesättigte oder ungesättigte Kohlenwasserstoffkette, die 12 bis 22 Kohlenstoffatome enthält und die gegebenenfalls mit einem oder mehreren Hydroxylsubstituenten substituiert ist, und X ein Glycerylrest ist;
als Wirkstoff zur Behandlung von Zeichen der Hautalterung durch selektive Beseitigung alternder Hautzellen.

## Revendications

1. Utilisation cosmétique d'un ou plusieurs monoglycérides de formule générale (I) :
RC(O)-X (I)
dans laquelle R est une chaîne hydrocarbonée linéaire ou ramifiée, saturée ou insaturée, qui contient de 12 à 22 atomes de carbone et qui est éventuellement substituée par un ou plusieurs substituants hydroxyle ; et X est un résidu glycéryle ; en tant que principe actif pour traiter des signes de vieillissement cutané par élimination sélective de cellules cutanées sénescentes.
